(19) Europäisches Patentamt
European Patent Office
Office européen des brevets

(11) Publication number: **0 528 452 A1**

(12) **EUROPEAN PATENT APPLICATION**

(21) Application number: **92201694.4**

(22) Date of filing: **11.06.92**

(51) Int. Cl.[5]: **C12N 9/10**, C12P 17/18, C07D 498/02

The applicant has filed a statement in accordance with Rule 28 (4) EPC (issue of a sample only to an expert). Accession number(s) of the deposit(s):ATCC 55087.

(30) Priority: **19.06.91 US 718451**

(43) Date of publication of application: **24.02.93 Bulletin 93/08**

(84) Designated Contracting States: **CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Shafiee, Ali**
**309 Orenda Circle**
**Westfield, NJ 07090(US)**
Inventor: **Colwell Jr., Lawrence F.**
**5 Princess Lane**
**Eatontown, NJ 07724(US)**
Inventor: **Kaplan, Louis**
**7 Lexington Road**
**New City, NY 10956(US)**
Inventor: **Arison, Byron H.**
**88 Century Lane**
**Watchung, NJ 07060(US)**
Inventor: **Dumont, Francis**
**54 West Cherry Street**
**Rahway, NJ 07065(US)**

(74) Representative: **Thompson, John Dr. et al**
**Merck & Co., Inc. European Patent**
**Department Terlings Park Eastwick Road**
**Harlow, Essex CM20 2OR (GB)**

(54) **Immunomycin enzymatic and/or microbial methylation products.**

(57) Described is a new enzyme, 31-O-desmethylimmunomycin O-methyltransferase (DIMT) which can specifically and preferentially methylate the C-31 hydroxy group in immunomycin type molecules, also designated FK520 and FR-900520 by Fujisawa. The enzyme can be extracted out of the microorganism Streptomyces hygroscopicus var. ascomyceticus, (Merck Culture Collection No. MA 6678) ATCC No. 55087. Employing the enzyme in its active form, supplemented with $Mg^{+2}$ ion, in the presence of the methyl donor, S-adenosyl methionine (SAM), a new FK520 (FR-900520) derivative has been prepared: 15-desmethyl FR-900520, made from 15,31-bisdesmethyl FK-900520. This new macrolide can act as an antagonist at moderately low dosages and reverse the immunosuppressant action of FK-506, and can be used diagnostically as a tool to determine the presence of FK-506 macrolide type immunosuppressants in natural product broths, distinguishing such immunosuppressants from other chemical families such as the cyclosporins. Also, it can act as an agonist at higher dosages and much lower dosages and is useful in preventing human host rejection of foreign organ transplants, e.g. bone marrow, liver, lung, kidney and heart transplants.

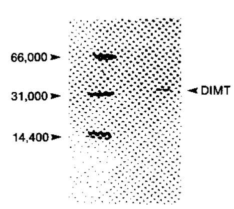


FIG. 4

EP 0 528 452 A1

Rank Xerox (UK) Business Services
(3.09/3.5x/3.0.1)

## BACKGROUND OF THE INVENTION

### 1. Field of the Invention

This invention relates to a new enzyme, 31-O-desmethylimmunomycin methyltransferase (DIMT) which can methylate the C-31 hydroxyl group in an FK-506 type molecule, e.g. 31-desmethylimmunomycin, and a new immunosuppressant, 15,31-bisdesmethylimmunomycin. Also disclosed are methods for their use in a human host for treatment of autoimmune diseases, infectious diseases and/or prevention of organ transplant rejections.

### 2. Brief Description of Disclosures in the Art

In 1983, the US FDA licensed cyclosporin, and extremely effective anti-rejection drug that revolutionized the field of organ transplant surgery. The drug acts by inhibiting the body's immune system from mobilizing its vast arsenal of natural protecting agents to reject the transplant's foreign protein.

As effective as the drug is in fighting transplantation rejection, it suffers drawbacks in causing kidney failure, liver damage and ulcers which in many cases can be very severe.

EPO Publication No. 0184162 to Fujisawa, hereby incorporated by reference, describes a new macrolide immunosuppressant FK-506 (FR-900506) which is reputed to be 100 times more effective than cyclosporin. The macrolide is produced by fermentation of a particular strain of Streptomyces tsukubaensis. Also described is the closely related macrolide immunosuppressant FK-900520 (FK-900520), produced by S. hygroscopicus subsp. yakushimaensis.

U.S. Patent 3,244,592 to T. Arai describes the culturing of Streptomyces hygroscopicus var. ascomyceticus to produce the antifungal "ascomycin".

What is desired in the art are newer immunosuppressants which are FK-506 like in behavior without its attendant side effects in addition to FK-506 antagonists which will substantially reverse the immunosuppressant effects of FK-506 to act as an antagonist and be useful in situations requiring the determination of FK-506 macrolide type activity as opposed to other chemical families such as the cyclosporins.

New protagonists and antagonists for FK-506 are constantly being searched for in the field.

### 3. Brief Description of the Figures

Figure 1 is an $^1$H NMR spectrum taken at 400 MHz of L-683,590 (immunomycin or FK-900520 or FR-900520) in $CDCl_3$ and showing its assigned molecular structure.

Figure 2 is an $^1$H NMR spectrum taken at 400 MHz of 15-31-bisdesmethylimmunomycin and its assigned molecular structure.

Figure 3 is an $^1$H NMR spectrum taken at 400 MHz of 15-desmethylimmunomycin and its assigned molecular structure.

Figure 4 is a SDS-PAGE chromatogram of DIMT illustrating a molecular weight by this technique of about 32,000.

## SUMMARY OF THE INVENTION

It has been found that a new enzyme, DIMT, can be isolated in substantially pure form from Streptomyces hygropscopicus var. ascomyceticus, (Merck Culture Collection No. MA 6678), ATCC No. 55087. The enzyme has a molecular weight of about 32,000 daltons, an isoelectric point 4.4, and is useful in being able to selectively methylate the C-31 hydroxy group in a 31-desmethyl FK-506 type molecule, in the presence of S-adenosyl methionine (SAM), a methyl donor agent.

By means of this enzyme, new C-31 methoxy FK-506 type compounds can be prepared for the first time and known C-31 methoxy compounds can be prepared by a novel methylation route specifically:

(1) starting with 13,31-bisdesmethylimmunomycin, described as L-683,756, the known C-13 desmethylimmunomycin, can be made by a different route. The known route is by biotransformation on FK-900520 by the microorganism MA 6474 (ATCC No. 53771).

(2) starting with the known 15,31-bisdesmethylimmunomycin, known as L-686,292, the novel 15-desmethyl immunomycin can be made.

(3) starting with the known 13,15,31-trisdesmethylimmunomycin, L-687,795, the novel 13,15-bisdesmethylimmunomycin can be made.

By this invention there is provided a composition comprising a purified cell free enzyme, DIMT, having a molecular weight of about 32 K daltons, as measured by SDS-PAGE, an isoelectric point (PI) of 4.4., and capable of catalyzing the C-31 O-methylation of a C-31 hydroxy containing FK-506 type molecule, in the presence of a methyl transfer agent, when supplemented with Mg $^{+}$2 ion.

Further provided is a process for methylating the C-31 hydroxy group in a C-31 hydroxy-containing FK-506 type molecule comprising the step of contacting the FK-506 type molecule with the methyl transfer agent S-adenosyl methionine in the presence of the DIMT enzyme described above, supplemented with the Mg $^{+}$2 ion, in an aqueous solvent therefor.

Also provided is a compound of the formula:

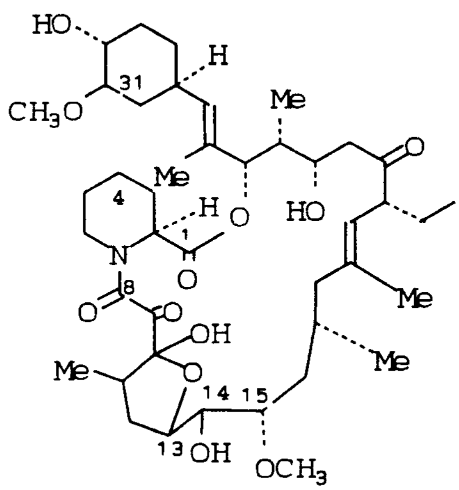

Structure VI

L-685, 487

C-13 desmethyl ring rearranged immunomycin

Further provided is a compound of the formula:

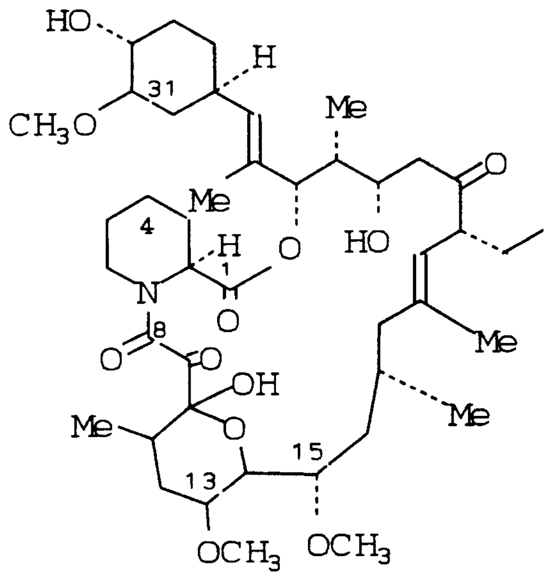

Structure I

FK-900520

FK-520

Immunomycin

L-683, 590

DETAILED DESCRIPTION OF THE INVENTION AND PREFERRED EMBODIMENTS

This invention is directed to a composition containing a purified cell free enzyme, DIMT, having a molecular weight of about 32 K daltons, as measured by SDS-PAGE, an isoelectric point (PI) of 4.4., and capable of catalyzing the C-31 O-methylaion of a C-31 hydroxy containing FK-506 type molecule, in the presence of a methyl transfer agent, when supplemented with $Mg^{+2}$ ion.

The enzyme can be isolated from the microorganism, Streptomyces hygroscopicus var. ascomyceticus, (Ma 6678) ATCC No. 55087 and purified for enzymatic use by the procedure described in Example 1.

The enzyme is useful in a process for methylating the C-31 hydroxy group in a C-31 hydroxy-containing FK-506 type molecule involving the step of contacting the FK-506 type molecule with the methyl transfer agent, e.g., S-adenosyl methionine, in the presence of the DIMT enzyme described, supplemented with $Mg^{+2}$ ion, in an aqueous solvent therefor.

Generally, the process is conducted at a pH from about 7-9 and in the temperature range of about 25-40°C.

The aqueous solvent system is generally a phosphate buffer of about pH = 7-8.

The Mg +2 ion is supplied as a soluble magnesium salt, e.g., magnesium chloride, magnesium sulfate, magnesium citrate, and the like.

Isolation and purification can be accomplished by conventional techniques including HPLC or reverse phase HPLC as described in the examples.

The enzyme is specific in methylating only the C-31 hydroxy group in an "FK-506 type molecule" and by that term is meant a molecule corresponding to the following Structure A, as described in PCT 89/05304 (June 15, 1989) and EP Publication 0,323,042 (July 5, 1989) to Fisons:

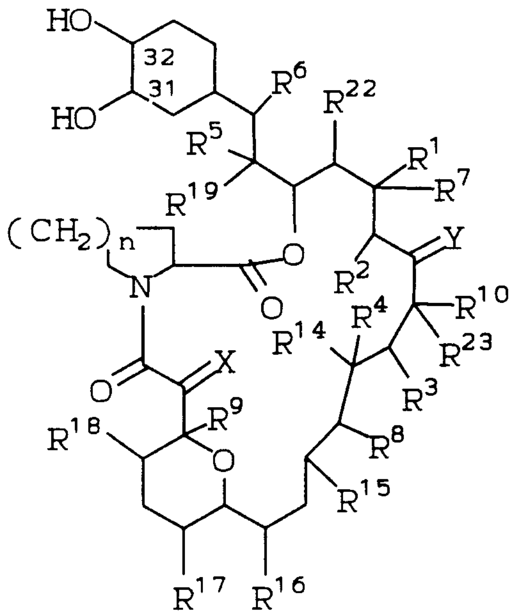

A

wherein each vicinal pair of substituents:

$R^1$ and $R^2$; $R^3$ and $R^4$; $R^5$ and $R^6$; independently:

a) represent two vicinal hydrogen atoms, or

b) form a second bond between the vicinal carbon atoms to which they are attached;

in addition to it significance above,

| | |
|---|---|
| $R^2$ | can represent an $C_1$-$C_{10}$ alkyl group; |
| $R^7$ | represents H, OH or O-$C_1$-$C_{10}$ alkyl, or in conjunction with $R^1$ it may represent =O; |
| $R^8$ and $R^9$ | independently represent H or OH; |
| $R^{10}$ | represents H; $C_1$-$C_{10}$ alkyl, wherein said alkyl can be substituted by one or more hydroxyl groups; $C_1$-$C_{10}$ alkenyl, which can be substituted by one or more hydroxyl groups, or $C_1$-$C_{10}$ alkyl substituted by =O; |
| X | represents O, (H, OH), (H,H) or -$CH_2O$-; |
| Y | represents O, (H, OH), (H,H), N-$NR^{11}R^{12}$ or N-$OR^{13}$ |

wherein,

| | |
|---|---|
| $R^{11}$ and $R^{12}$ | independently represent H, $C_1$-$C_{10}$alkyl, $C_1$-$C_{10}$aryl or tosyl, and |
| $R^{13}$, $R^{14}$, $R^{15}$, $R^{16}$, $R^{17}$, $R^{18}$, $R^{19}$, $R^{22}$ and $R^{23}$ | independently represent H or $C_1$-$C_{10}$ alkyl; |
| | n is 1,2, or 3; |

in addition to their significances above, Y, $R^{10}$ and $R^{23}$, together with the carbon atoms to which they are attached, can represent a 5- or 6- membered N-, S- O-containing heterocyclic ring, which is saturated or unsaturated, and which can be substituted by one or more groups selected from $C_1$-$C_{10}$ alkyl, hydroxyl, $C_1$-$C_{10}$ alkyl substituted by one or more hydroxyl groups, O-$C_1$-$C_{10}$ alkyl, benzyl and -$CH_2Se(C_6H_5)$; provided that when X and Y both represent O; $R^9$ represents OH; $R^{14}$, $R^{15}$, $R^{16}$, $R^{17}$, $R^{18}$, $R^{19}$ and $R^{22}$ each represent methyl; $R^8$ and $R^{23}$ each represent H; [$R^3$ and $R^4$] and [$R^5$ and $R^6$] each represent a carbon-carbon bond; and pharmaceutically acceptable salts thereof, which includes acid addition salts of any amine groups present.

Also included are specific FK-506 structures corresponding to Structure B, which are published in U.S. Patent 4,894,366 (January 16, 1990) to Fujisawa:

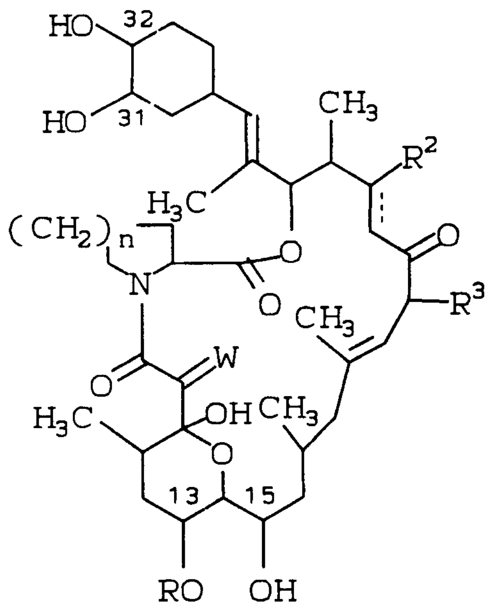

B

wherein:

W represents O, (H, OH), (H, H);

R is H, $CH_3$;

$R^2$ is hydrogen, hydroxy or lower alkanoyloxy,

$R^3$ is methyl, ethyl, propyl or allyl,

n is an integer of 1 or 2, and the symbol of a line and dotted line is a single bond or a double bond,

and a pharmaceutically acceptable basic salt thereof.

Specifically preferred are the compounds where:

R is $CH_3$

$R^2$ is hydroxy;

$R^3$ is ethyl;

n is 2; and

R is H;

$R_2$ is hydroxy;

$R_3$ is ethyl;

n is 2.

Particularly preferred is the C-31 methylation process wherein said starting FK-506 type compound is of the formula:

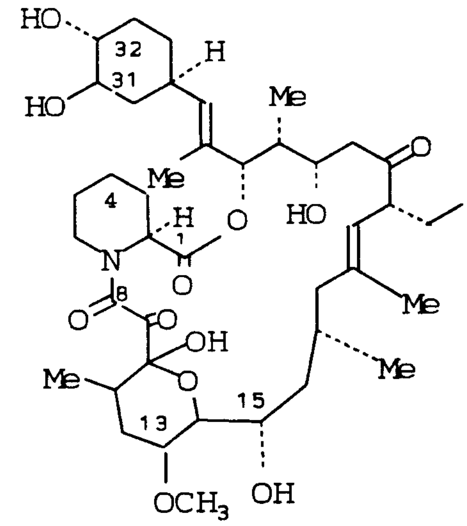

L-686, 292

IV

and the methylated product is a compound of the formula:

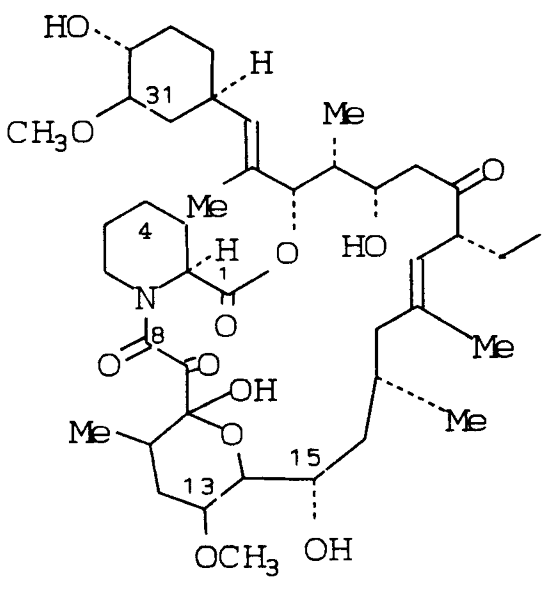

VII

Also preferred is the process wherein said starting FK-506 type compound is of the formula:

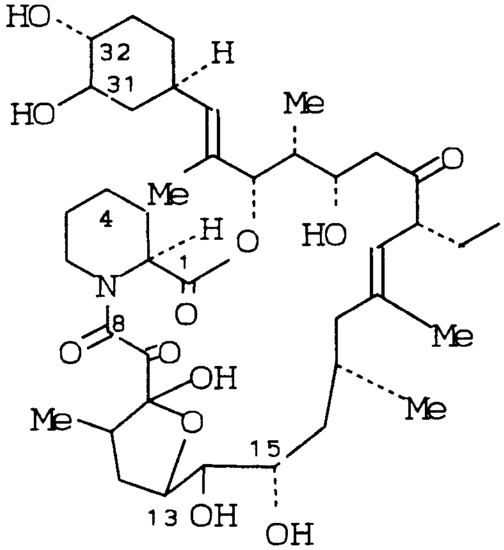

L-687, 795

V,

and the product is a Compound of the formula:

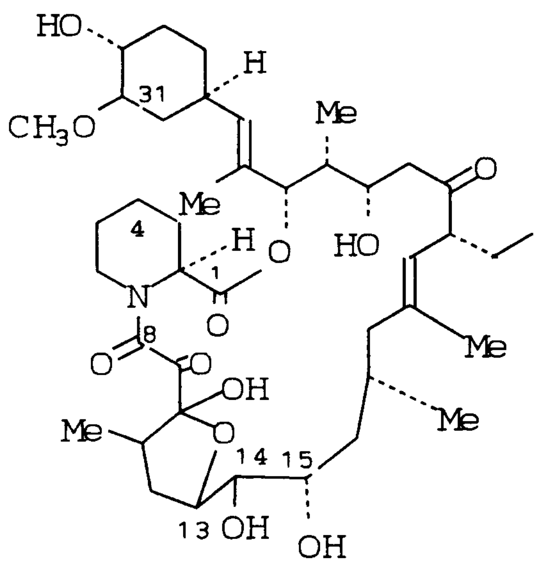

VIII

The resultant C-31 methylated compounds can exhibit immunosuppressive activity, i.e., positive inhibition of T-cell activation, as demonstrated by the calcium ionophore (ionomycin) plus phorbol myristate acetate (PMA) induced T-cell stimulation assay, also referred to herein as the "T-cell proliferation assay". The principle of this assay is to measure the proliferation of mouse T lymphocytes stimulated with the combination of ionomycin plus PMA. A positive sample in this assay will inhibit T-cell proliferation, as indicated by reduced tritiated thymidine uptake.

The process of the present invention involves the incubation and extraction of Streptomyces hygroscopicus subsp. ascomyceticus, Merck Culture Collection No. MA 6678, ATCC No. 55087 to isolate the DIMT enzyme. The microorganism is currently on deposit with the American Type Culture Collection, 12301 Parklawn Drive in Rockville, Maryland as ATCC No. 55087, and in the Merck Culture Collection in Rahway,

New Jersey as MA 6678. The physical characteristics and taxonomy, including morphological, cultural, biological and physiological characteristics of the microorganism, which is derived from parent strain MA 6475, ATCC No. 14891, are briefly described hereinbelow.

The following is a general description of Streptomyces hygroscopicus subsp. ascomyceticus strain MA6678, ATCC 55087 and the parent culture MA6475, ATCC 14891. Observations of growth, general cultural characteristics and carbon source utilization were made in accordance with the methods of Shirling and Gottlieb (Internat. J. System. Bacteriol. 16: 313 - 340). Chemical composition of the cells was determined using the methods of Lechevalier and Lechevalier (in Actinomycete Taxonomy, A. Dietz and D.W. Thayer, Ed. Society for Industrial Microbiology, 1980). Coloration of the culture was determined by comparison with color standards Centroid Color Charts (US Dept. of Commerce National Bureau of Standards Centroid Color Charts (US Dept. of Commerce National Bureau of Standards supplement to NBS Circular 553, 1985).

**Source** - Strain MA6678 (ATCC 55087) was derived from MA6475 (ATCC 14891), Streptomyces hygroscopicus subsp ascomyceticus.

**Analysis of Cell wall Composition** - The peptidoglycan of strains MA6678 and MA6475 both contain L-diaminopimelic acid.

**General growth characteristics** - Both cultures are found to grow well on Yeast Malt Extract, Glycerol Asparagine, Inorganic Salts-Starch, Oatmeal, and Trypticase Soy agars. Growth occurs at 27°C and 37°C. Both cultures also grow well in liquid media such as Yeast Dextrose broth.

**Colony morphology** (on Yeast Malt Extract Agar) MA6678 - Substrate mycelium is brilliant yellow (83 brill Y) and colonies are opaque, raised, erose and rubbery. The colony surface is powdery to rough textured. Aerial mycelia appear after 7 days incubation and are white in color (263 White). Spore mass, when present, is white (263 White) and turns to black (267 Black) upon prolonged incubation.

MA6475 - Substrate mycelium is medium yellow (87 m.Y) and colonies are opaque, raised, erose and rubbery.

The colony surface is powdery. Aerial mycelia appear by 14 days and are medium gray (265 med Gray). Spore mass, when present is medium gray (265 med Gray) and turns black (267 Black) upon prolonged incubation.

**Micromorphology MA6678 and MA6475**

Aerial mycelium (0.57 - 0.76 $\mu$m dia.) arises from a substrate mycelium and is branched and flexous. In mature cultures, the aerial mycelium commonly terminates in spores borne predominantly in spiral chains. Spore mass tends to coalesce into an amorphous mass by 21 days.

| Yeast Extract-Malt Extract | | |
|---|---|---|
| | MA6475 | MA6678 |
| Amount of Growth:<br>Aerial Mycelium:<br><br>Soluble Pigments:<br>Substrate Mycelium:<br>Brilliant yellow | Good<br>Medium gray (265 med. Gray) branched spiral sporophores<br>None<br><br><br>(87 m.Y) | Good<br>White (263 White) branched, spiral sporophores<br><br>None<br>Medium yellow<br><br>(83 brill Y) |

| Glucose Asparagine | | |
|---|---|---|
| | MA6475 | MA6678 |
| Amount of Growth:<br>Aerial Mycelium:<br><br>Soluble Pigments:<br>Substrate Mycelium:<br>Light yellow | Good<br>Medium gray (265 med. Gray)<br>branched spiral sporophores<br>None<br><br><br>(89 p.Y) | sparse<br>Yellow white (92 y. White)<br>branched, spiral sporophores<br>None<br>Pale yellow<br><br>(86 1.Y) |

| Inorganic Salts Starch | | |
|---|---|---|
| | MA6475 | MA6678 |
| Amount of Growth:<br>Aerial Mycelium:<br><br>Soluble Pigments:<br>Substrate Mycelium:<br>Pale yellow | Good<br>Medium gray (265 med. Gray)<br>branched spiral sporophores<br>None<br><br><br>(89 p.Y) | Good<br>Yellow white (92 y. White)<br>branched, spiral sporophores<br>None<br>Pale yellow<br><br>(89 p.Y) |

| Oatmeal | | |
|---|---|---|
| | MA6475 | MA6678 |
| Amount of Growth:<br>Aerial Mycelium:<br><br>Soluble Pigments:<br>Substrate Mycelium:<br>Pale yellow | Good<br>Dark gray (265 d. Gray)<br>branched spiral sporophores<br>None<br><br><br>(90 gy.Y) | Good<br>White (263 White) branched, spiral sporophores<br><br>None<br>Gray yellow<br><br>(89 p.Y) |

| Sigma Water | | |
|---|---|---|
| | MA6475 | MA6678 |
| Amount of Growth:<br>Aerial Mycelium:<br>Soluble Pigments:<br>Substrate Mycelium: | Fair<br>Black (267 Black) edges, spiral sporophores<br>None<br>---- | Sparse<br>----<br>None<br>---- |

| Czapek | | |
|---|---|---|
| | MA6475 | MA6678 |
| Amount of Growth: | Good | Good |
| Aerial Mycelium: | Light gray (264 1. Gray) branched spiral sporophores | White (263 White) branched, spiral sporophores |
| Soluble Pigments: | None | None |
| Substrate Mycelium: | | Medium gray |
| Yellow white | (265 med Gy) | (92 y White) |

| Peptone Iron | | |
|---|---|---|
| | MA6475 | MA6678 |
| Amount of Growth: | Good | Good |
| Aerial Mycelium: | ---- | ---- |
| Soluble Pigments: | melanin negative | melanin negative |
| Substrate Mycelium: | ---- | ---- |

**Miscellaneous physiological reactions**

MA6678 - Culture does not produce melanoid pigments in tryptone yeast extract broth, starch is hydrolyzed, yellow, non-pH dependant diffusible pigment produced on Pridham-Gottlieb basal medium supplemented with 1% cellobiose, D-fructose or D-mannose. Carbon source utilization pattern is as follows: good utilization of cellobiose, D-fructose, α-D-lactose, β-D-lactose, D-maltose, D-mannitol, D-mannose, or L-rhamnose; poor utilization of L-arabinose; no utilization of D-arabinose, inositol, D-raffinose, sucrose, D-xylose, or L-xylose.

MA6475 - Culture does not produce melanoid pigments in tryptone yeast extract broth, starch is hydrolyzed. Carbon source utilization pattern is as follows: good utilization of cellobiose, D-fructose, α-D-lactose, β-D-lactose, D-maltose, D-mannitol, D-mannose, L-rhamnose or D-xylose; poor utilization of L-arabinose or inositol; no utilization of D-arabinose, D-raffinose, sucrose, or L-xylose.

**Diagnosis** - The chemotaxonomic and morphological characteristics of strain MA6678 compare favorably with those of the parent strain MA6475. Phenotypic characteristics which serve to differentiate the MA6678 from the parent strain include: the production of a soluble yellow pigment on some media and the failure to utilize inositol or D-xylose as a sole carbon source. It should be pointed out that the specific epithet Streptomyces hygroscopicus (and hence all subspecies) is currently considered to be a subjective synonym of Streptomyces violaceusniger (Bergey's Manual of Systematic Bacteriology, Vol 4, 1989).

| Carbohydrate utilization pattern of Streptomyces hygroscopicus subsp. ascomyceticus strains MA6475 and MA6678 at 21 days | | |
|---|---|---|
| Carbon Source | Utilization by MA6475 | Utilization by MA6678 |
| D-arabinose | 0 | 0 |
| L-arabinose | 1 | 1 |
| cellobiose | 2 | 2 |
| D-fructose | 2 | 2 |
| inositol | 1 | 0 |
| α-D-lactose | 2 | 2 |
| β-D-lactose | 2 | 2 |
| D-maltose | 2 | 2 |
| D-mannitol | 2 | 2 |
| D-mannose | 2 | 2 |
| D-raffinose | 0 | 0 |
| L-rhamnose | 2 | 2 |
| sucrose | 0 | 0 |
| D-xylose | 2 | 0 |
| L-xylose | 0 | 0 |
| a-D-glucose (control) | 2 | 3 |
| 3 = good utilization, 2 = moderate utilization, 1 = poor utilization 0 = no utilization | | |

The present invention process can be practiced with any "DIMT-producing" strain of Streptomyces, including (MA 6475) ATCC No. 14891, and particularly preferred is the ATCC No. 55087 strain.

The L-683,590 (FK-900520) starting material used in the Examples, can be obtained by the fermentation of S. hygroscopicus var. ascomyceticus, ATCC No. 14891, as described in U.S. Patent 3,244,592, and by the fermentation of S. hygroscopicus subsp. yakushimaensis No. 7278, (to produce FR-900520, or "FK-900520", which is identical to L-683,590) as described in EPO Publication No. 0184162 to Fujisawa.

It is known that the FK-506 antagonist, L-686,292, can be obtained by the incubation of the microorganism Actinoplanacete sp. (MA 6559), ATCC No. 53771, in the presence of the macrolide immunosuppressant FK-900520, under submerged aerobic conditions in an aqueous carbohydrate medium, containing a nitrogen nutrient, said conditions being conducted at a pH of about 7 for a sufficient time to selectively C-15, C-31 bisdemethylate FK-900520, i.e., remove two methyl radicals from two different methoxyl groups. L-686,292 is a minor fermentation product found in the broth along with L-687,795, the C-13, C-15, C-31 trisdemethylated analog of L-683,590, disclosed in copending Serial No. 348,243, corresponding to EP-A-0396399. Major components in the broth include L-683,756, a C-13, C-31 bisdemethylated ring rearranged derivative of L-683,590 as described in Serial No. 297,630, corresponding to EP-A-0378320, and L-683,742, the monodemethylated analog of L-683,590, same assignee, and L-683,756, the C-13, C-31 bisdemethylated analog of L-683,590, disclosed in Serial No. 297,630, corresponding to EP-A-0378320.

The produced Structures VII and VIII can be recovered from the enzymatic methylation medium by conventional means which are commonly used for the recovery of other known biologically active substances. The Structure VII and VIII substances produced can be isolated and purified from the filtrate, by a conventional method such as concentration under reduced pressure, lyophilization, extraction with a conventional solvent, such as methanol and the like, pH adjustment, treatment with a conventional resin (e.g. anion or cation exchange resin, non-ionic adsorption resin, etc.), treatment with a conventional adsorbent (e.g. activated charcoal, silicic acid, silica gel, cellulose, alumina, etc.), crystallization, recrystallization, and the like. A preferred method is solvent extraction, particularly using methanol.

The product Structure VIII exhibits positive immunosuppressive activity by the "T-cell proliferation assay" and possesses utility on this basis. The product Structure VII exhibits antagonist properties to FK-506 and is useful in situations requiring an antidote to an FK-506 overdose.

The Structures VII and VIII obtained according to the processes as explained above can be isolated and purified in a conventional manner, for example, extraction, precipitation, fractional crystallization, recrystallization, chromatography, and the like.

Suitable formulations of the materials may also include conventional pharmaceutically acceptable biolabile esters of VII and VIII, formed via the hydroxy groups on the molecule, such as the acetate.

It is to be noted that the tautomeric and conformational isomer(s) of VII and VIII, including those due to rearrangement of the hemiketal ring system are also included within the scope of the present invention.

The Structure VIII of the present invention possesses pharmacological activity such as immunosuppressive activity, antimicrobial activity, and the like, and therefore are useful for the treatment and prevention of the transplantation rejection of organs or tissues such as heart, kidney, liver, medulla ossium, skin, etc., graft-versus-host diseases by medulla ossium transplantation, autoimmune diseases such as rheumatoid arthritis, systemic lupus erythematosus, Hashimoto's thyroiditis, multiple sclerosis, myasthenia gravis, type I diabetes, uveitis, and the like.

The pharmaceutical composition of this invention can be used in the form of a pharmaceutical preparation, for example, in solid, semisolid or liquid form, which contains the Structure VII or VIII, of the present invention, as an active ingredient, in admixture with an organic or inorganic carrier or excipient suitable for external, enteral or parenteral applications. The active ingredient may be compounded, for example, with the usual non-toxic, pharmaceutically acceptable carriers for tablets, pellets, capsules, suppositories, solutions, emulsions, suspensions, and any other form suitable for use. The carriers which can be used are water, glucose, lactose, gum acacia, gelatin, mannitol, starch paste, magnesium trisilicate, talc, corn starch, keratin, colloidal silica, potato starch, urea and other carriers suitable for use in manufacturing preparations, in solid, semisolid, or liquid form, and in addition auxiliary, stabilizing, thickening and coloring agents and perfumes may be used. The active object compound is included in the pharmaceutical composition in an amount sufficient to produce the desired effect upon the process or condition of diseases.

For applying this composition to a human, it is preferable to apply if by parenteral or enteral administration. While the dosage of therapeutically effective amount of the Structure VIII, varies from, and also depends upon the age and condition of each individual patient to be treated, a daily dose at least equivalent to the amount of FK-506 administered, or FK-906 type macrolide sufficient to reverse the effects of the FK-506 type immunosuppressive (calculated on the basis of a 70 kg man) of about 0.01-1000 mg, preferably 0.1-500 mg and more preferably 0.5-100 mg, of the active ingredient is generally given for treating diseases, and an average single dose of about 6.5 mg, 1 mg, will be sufficient.

Structures of the compounds involved in this invention include the following:

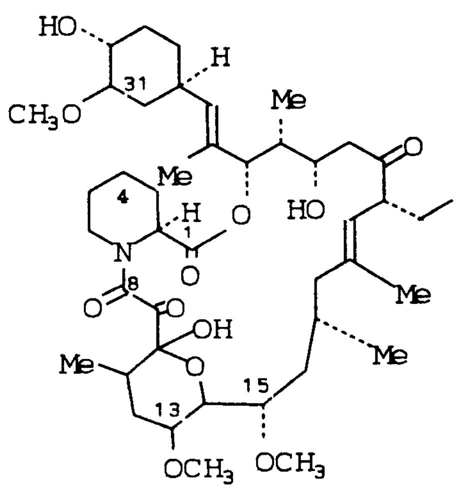

Structure I

FR-900520

FK-520

Immunomycin

L-683, 590

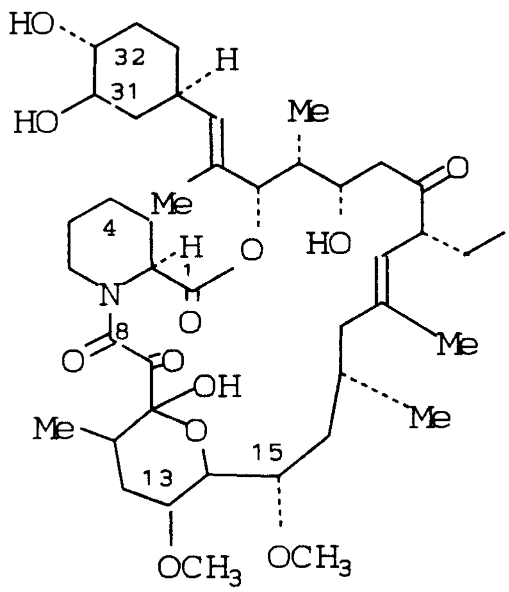

Structure II

L-683,742

C-31 desmethylimmunomycin

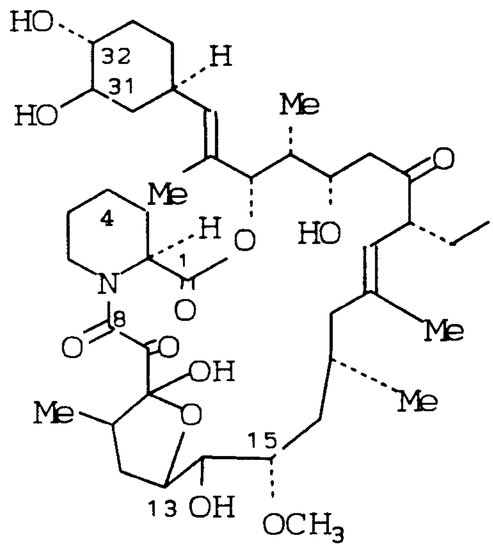

Structure III

L-683,756

C-13, C-31 bisdemethyl ring rearranged immunomycin

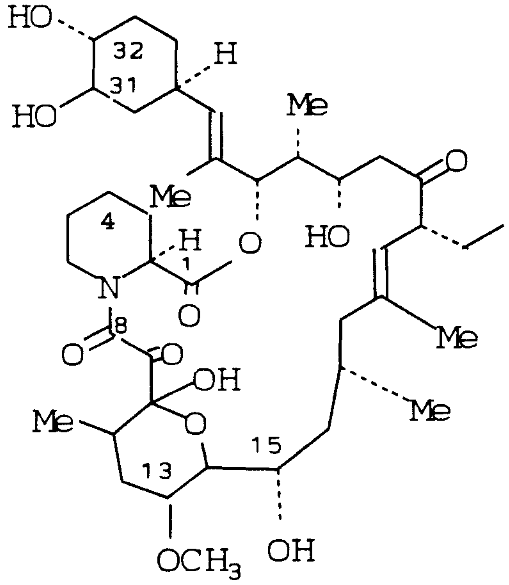

Structure IV

L-686,292

C-15, C-31 bisdemethyl-immunomycin

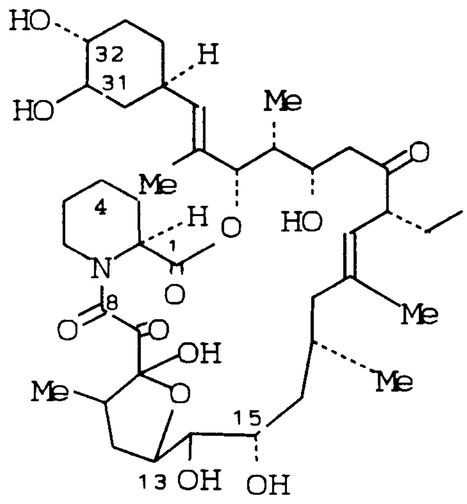

Structure V

L-687,795

C-13, C-15, C-31 trisdemethyl ring rearranged immunomycin

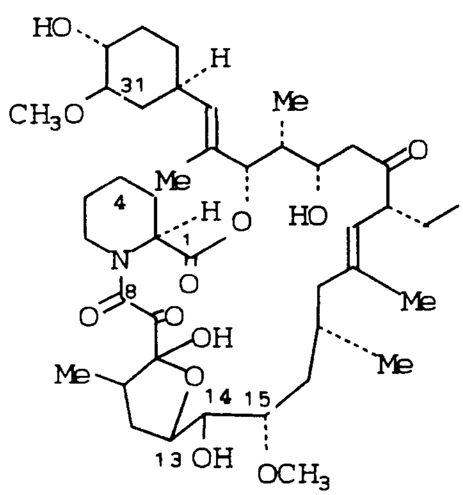

Structure VI

L-685,487

C-13 desmethyl ring rearranged immunomycin

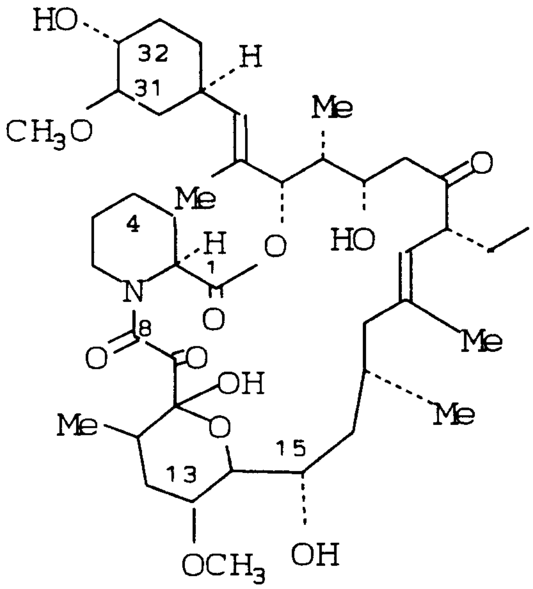

Structure VII

C-15 desmethylimmunomycin

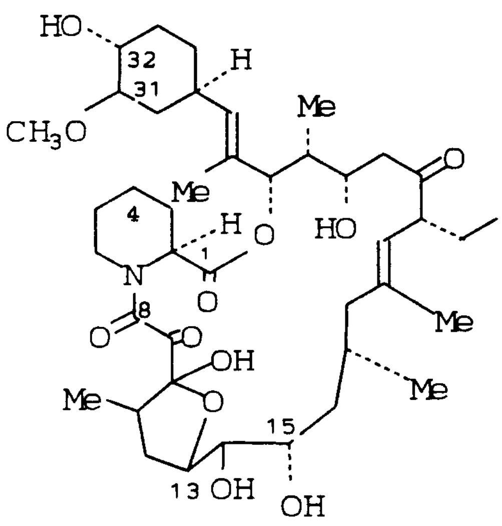

Structure VIII

C-13, C-15 bisdesmethyl ring rearranged immunomycin

EXAMPLE 1

**Isolation and characterization of S-adenosyl-L-methionine: 31-O-desmethylimmunomycin O-methyl-transferase.**

Immunomycin (Structure I) is a new macrocyclic lactone/lactam antibiotic with effective immunosuppressive and antifungal activities (See EP Publication No. 0323865 (1989) by F.J. Dumont, et al.). Precursor feeding studies in this laboratory has established the polyketide nature of the macrocyclic structure. Furthermore, the data from the feeding studies indicate that the three methyl groups at positions 13, 15 and 31 originate from methionine through S-adenosyl-L-methionine (SAM). With the development of a mutant strain of S. hygroscopicus var. ascomyceticus (MA 6674) which produces 31-O-desmethylimmunomycin, it has become possible to isolate and characterize the enzyme, S-adenosyl-L-methionine: 31-O-desmethylimmunomycin O-methyltransferase (DIMT).

**Experimental**

**Bacterial growth and mycelium preparation.**

Frozen vegetative mycelium of Streptomyces hygroscopicus var. ascomyceticus (ATCC 55087) was cultured in the seed and fermentation media. Mycelial cells were harvested at 72 hours and washed three times with 50mM phosphate buffer, pH 7.5, containing 0.5M KCl and finally with the same buffer with no KCl. Washed mycelium was used for the preparation of the cell-free extract.

**Time course of the mycelial growth and immunomycin production.**

At different time intervals, an aliquot of the fermentation culture was centrifuged at 2000 rpm for 10 mins. and packed cell volume was determined. Similarly, an aliquot was extracted with methanol and immunomycin production was quantitated by HPLC.

**S-adenosyl-L-methionine: 31-O-desmethylimmunomycin O-methyltransferase (DIMT) assay and product identification.**

The assay was carried out in 1ml. mixture containing 0.025 mM 31-desmethylimmunomycin, 1mM $MgSO_4$ and different quantities of the enzyme source in 50mM phosphate buffer, pH 7.5. Reaction was initiated by the addition of 1 nmole of 14C-SAM (S-adenosyl-L-[methyl-14C] methionine with the specific activity of 46mCi/mmole. Incubation of the complete mixture was carried out at 34° C for 20 mins. and the reaction was terminated by the addition of ethyl acetate. Product of the reaction was extracted with 2ml. of ethyl acetate and 1ml. of the extract was used for TLC and HPLC analysis. For the analysis of the radioactive reaction product (immunomycin), the ethyl acetate extract was spiked with the standard immunomycin and subjected to the TLC with plate having plastic support. The plastic sheet was developed in chloroform:methanol (9:1) and area showing standard immunomycin under UV-light was cut and the radioactivity in the cut-strip was measured. The amount of the product formed was calculated from the measured radioactivity. For the HPLC analysis of the product of the enzyme reaction, several enzymatic reaction mixtures were pooled and the extract was subjected to the TLC analysis as above. The area on the silica gel plate showing the band having Rf value identical with the standard immunomycin was scraped and eluted with ethyl acetate. The ethyl acetate extract was washed with water, dried under nitrogen and the residue was subjected to the HPLC analysis on Partisil 5-ODS-3 column with acetonitrile:water: phosphoric acid (65: 35: 0.1) as the eluting solvent. One milliliter fractions were collected and a portion of each fraction was examined for the radioactivity.

**Isolation of the 31-O-methyltransferase (DIMT).**

(All of the procedures were carried out at 4° C in the cold room or on ice). Washed mycelium was suspended in 50mM phosphate buffer, pH 7.5 containing 1mM PMSF and 1mg/ml of lysozyme. The resulting suspension was stirred overnight and centrifuged at 15000 rpm for 30 mins. The pellet thus obtained was sonicated for 2' at 30" intervals by microtip-equipped sonifier. The cell homogenate was centrifuged as before and the supernatants were combined and re-centrifuged at 105K x g for 45 mins. The supernatant was then brought to 1% streptomycin sulfate, stirred for 30 mins. and centrifuged at 105K x g

as before. The resulting supernatant, designated as the crude extract, was made to contain 0.1mM S-adenosyl-L-methionine (SAM) and 10% ethanol following the buffer system suggested for the isolation of the methyltransferases from Streptomyces fradiae, a tylosin-producing organism (See N. J. Bauern. et al., J.B.C. Vol. 263, p. 15619 (1988).) The crude extract was then brought to 30% ammonium sulfate saturation and after 30 mins. stirring was centrifuged at 15000 rpm for 30 mins. The resulting supernatant was brought to 60% ammonium sulfate saturation and after identical stirring and centrifugation procedures, its precipitate was recovered. This fraction, designated as 30-60% cut, was dialyzed against 50mM phosphate buffer, pH 7.5, containing 1mM PMSF, 0.1mM SAM and 10% ethanol (Buffer L) using PD-10 Pharmacia column. This fraction was chromatographed on a DEAE-Sepharose containing column. This column was eluted stepwise with buffer L containing various concentration of KCl. The DIMT activity was completely eluted with the buffer containing 0.3M KCl. The DIMT active fraction was dialyzed against buffer L and applied on the preparative MonoQ HR10/10-FPLC column previously equilibrated with the same buffer. One milliliter fractions, eluted under a linear gradient from zero to 1M KCl with a 130 minute duration in buffer L with a flow rate of 60ml/hr, were collected and examined for the DIMT activity. Fractions, showing the DIMT activity were pooled and after the necessary treatment was subjected to an analytical MonoQ HR5/5-FPLC column. This column was developed as above except for the KCL concentration which was 0.5M. Fractions collected from the analytical MonoQ column was examined for the DIMT activity and active fractions were pooled. and subjected to chromatofocusing. Chromatofocusing was carried out on a MonoP HR5/20 column with pH interval of 7-4 on Pharmacia FPLC system. Column was developed with the polybuffer and 1ml. fractions with a flow rate of 30ml. an hour were collected. pH of the fractions were adjusted to 7.2 immediately after elution and pH determination. Enzyme assay was carried out on various fractions and peak DIMT activity was located in fraction 35. This fraction was then applied on a Superose-12 column. This column was eluted with buffer L containing 150mM NaCl with a flow rate of 18ml./hr. and 0.3ml. fractions were collected. The fraction having the peak DIMT activity was finally subjected to a 12.5% native polyacrylamide electrophoresis (native-PAGE). After electrophoresis, cuts were made across the face of the gel and each gel cut was eluted by diffusion and assayed for the DIMT activity. The DIMT activity was located in the fraction eluted from the gel cut with Rf value higher than Rf value of the standard egg albumin in the same gel.

**General properties of DIMT.**

Examination of the optimal temperature, pH and metal requirement of the DIMT activity was carried out on the enzyme preparation obtained from DEAE-Sepharose column. For kinetic analysis, a preparation which had been purified through a second MonoQ column chromatography was utilized.

**Molecular weight and isoelectric point determination of DIMT.**

The molecular weight of the native enzyme was determined by gel filtration chromatography on Superose-12 column. Column was equilibrated and run in buffer L containing 150mM NaCl and with a flow rate of 18ml./hour. The apparent molecular weight of the denatured enzyme was estimated by SDS-PAGE according to Laemmli (Nature (London) Vol. 227, pp. 680-685 (1970)). In both gel filtration and SDS-PAGE, bovine serum albumin (Mr = 66000), egg albumin (Mr = 45000), carbonic anhydrase (Mr = 31000), and cytochrome C (Mr = 12400) were used as standards. Isoelectric point of the purified enzyme was estimated by both chromatofocusing on a Pharmacia MonoP HR5/20 column with a pH interval of 7-4 and calibrated isoelectric focusing-PAGE having interval of 3-9.

**General analytical methods.**

Native-PAGE was carried out according to Laemmli, supra, with the elimination of the SDS from the buffer system. Protein concentration was determined by the Bio-Rad protein assay system with bovine serum albumin as standard.

**Results and Discussion**

**Time course of the mycelial growth and immunomycin production.**

Packed cell volume and immunomycin production, as a function of time, were determined as an indicator for the titer of the immunomycin biosynthetic enzymes. Both the mycelial mass and immunomycin

production peaked between 72 to 95 hours into the age of the cultures. It was therefore decided to use 72 hour old mycelium for the isolation of the DIMT enzyme.

**Properties and localization of DIMT.**

Initially, using the crude extract, enzyme assay was established based on the assay system previously reported for the S-adenosyl-L-methionine-dependent methylation of tylosin and avermectin (See Bauern, N.J. et al., J. Biol. Chem. 263, p. 15619 (1988) and Schulman, M.D. et al., Antimicrobial Agents Chemotherapeutics, 29, p. 620 (1989 ). This preparation showed an absolute requirement for SAM and magnesium ions and EDTA completely inhibited the activity. The enzyme activity was also inhibited by ammonium sulfate, which was reversed after complete dialysis. Optimal reaction temperature and pH were also established to be 35°C and 8.5, respectively. Using the optimal condition, the enzyme activity could only be detected in the cell-free extract (crude extract) obtained after the 105K x g centrifugation step. No enhancement of the activity was observed upon reconstitution of the pellet with the crude extract.

**Isolation and purification of DIMT.**

Using optimized assay condition, a 72-hour old mycelial cells provided us with a cell-free preparation which was functional with regard to DIMT activity. The fraction containing this activity was isolated to a high degree of purity after a series of purification steps which included ammonium sulfate precipitation, DEAE-Sepharose, preparative and analytical MonoQ, chromatofocusing and Superose-12 column chromatographies. The elution profiles and DIMT activity of the fractions from preparative MonoQ, analytical MonoQ, chromatofocusing P and Superose-12 columns were determined. The DIMT was consistently eluted in a single peak with increasing specific activity. High degree of purity of of the DIMT was finally achieved when a highly purified fraction from Superose-12 column was subjected to native-PAGE. Fraction which was eluted from the native polyacrylamide gel after electrophoresis was enzymatically active and showed a single band on SDS-PAGE (See Figure 4) and isoelectric focusing gel.

**Molecular weight and isoelectric focusing point.**

Molecular weight of the purified DIMT was determined by SDS-PAGE and Superose-12 column chromatography. From the calibrated SDS-PAGE the purified DIMT showed a single homogeneous band with an apparent molecular weight of about 32,000 (See Figure 4). This preparation was also symmetrically eluted from a calibrated Superose-12 column with an elution volume identical to that of a standard carbonic anhydrase (Mr = 31000), therefore indicating an apparent molecular weight of 31000 for the native enzyme. Isoelectric point of the purified DIMT was estimated by both chromatofocusing and calibrated isoelectric focusing-PAGE. Both systems demonstrated an isoelectric point of 4.4 for the native enzyme.

**S-adenosyl-L-methionine (SAM) dependency and magnesium ion requirement**. DIMT requires SAM and $Mg^{++}$ for activity. As found, there is also major inhibition of DIMT activity by EDTA, sinefungin and S-adenosyl-homocysteine (SAH), reconfirming the SAM and $Mg^{++}$ requirement for this enzyme.

**pH and temperature dependencies of DIMT**. The optimum activity of DIMT was seen at ph 8.5 and a temperature of 35°C.

Kinetic studies. With the established incubation conditions, the enzymic velocity was directly proportional to the amount of protein up to about 60 ug. Under similar condition, when 25 ug of the protein was incubated for different period of time, the initial velocity was maintained for 30 mins. Under the condition for measurement of the initial velocity, the apparent Km and Vmax of DIMT were determined with 31-O-desmethylimmunomycin and SAM. The kinetic utilization of 31-O-desmethylimmunomycin was determined with 40uM SAM, while the kinetic utilization of SAM was examined with 25uM of 31-O-desmethylimmunomycin as cosubstrate. The apparent Km and Vmax of DIMT for 31-O-desmethylimmumomycin and SAM were determined from the Lineweaver-Burk reciprocal plots. The graphically obtained kinetic parameters indicate values of 45.5 nmole/hr/mg and 11uM for apparent Vmax and Km, respectively, when 31-O-desmethylimmunomycin was the variable substrate. Similarly, values of 100 nmole/hr/mg and 12.5uM were estimated for Vmax and Km when SAM was utilized as the variable substrate.

EXAMPLE 2

SYNTHESIS OF 13,31-BISDESMETHYL RING REARRANGED FK-900520 (L-683,756)-Structure III

Microorganism and Culture Conditions

The lyophilized culture (MA 6559) ATCC No. 53771 was used to inoculate a 250 ml baffled shake flask containing 50 ml of autoclaved (sterilized) seed medium A consisting of (in units of grams/liter) dextrin 10.0%, dextrose 1.0%, beef extract 3.0%, ardamine PH (Yeast Products, Inc.) 5.0%, N-Z Amine type E $5.0O_4.7H_20$ 0.05%, $KH_2PO_4$ 0.37%, and $CaCO_3$ 0.5%. The pH of the seed medium was adjusted to 7.1 before autoclaving. The seed was incubated in the seed medium at 27° C for 24 hours on a rotary shaker operating at 220 rpm.

Alternatively, when frozen vegetative mycelia or a slant source is used, the culture is incubated in the seed medium at 27° C for 24 hours at 220 rpm. A 2.5 ml aliquot of the resulting seed medium was used to inoculate a 250 ml non-baffled shake flask containing 50 ml of autoclaved (sterilized) production media B.

FK-900520 was added as a solution in dimethylsulfoxide to achieve a final concentration of 0.1 mg/ml concentration. The shake flask contents were subsequently incubated for 24 hours at 27° C on a rotary shaker operating at 220 rpm, and extracted by the following procedure described below.

Isolation and Purification Procedure for Each Broth

The whole broth (100 ml) of transformation media B was extracted three times with methylene chloride (3 x 100 ml). Methylene chloride extracts were combined, dried over sodium sulfate, and concentrated under vacuum to an oily residue. The residue was dissolved in acetonitrile and subjected to high performance liquid chromatography (HPLC) purification.

HPLC was carried out on Whatman Partisil 10 ODS-3, 9.4 mm x 25 cm column and monitored at 205 nm and 225 nm at 60° C. The column was developed at 3 ml/min. with a linear gradient from 0.1% aqueous $H_3PO_4$-$CH_3CN$, 45:55 to 0.1% aqueous $H_3PO_4$-$CH_3CN$, 20:80 in 40 minutes. The compound was collected during repeated injections of the above described extract. The fractions at retention time 11.5 minutes were pooled, adjusted to pH 6.5 and evaporated to remove acetonitrile. The compound was further purified using a $C_{18}$ Sep-Pak (Waters Associates) and acetonitrile-water elution solvent to yield 1.2 mg. The compound was designated as L-682,992. If L-682,993 is present, it will have a longer retention time than L-682,992 since it is monodemethylated and thus less polar.

Characterization

L-683,756 was characterized via NMR spectrometry. The assigned molecular structure is shown as Structure III.

EXAMPLE 3

SYNTHESIS OF 15,31-BISDESMETHYL FK-900520 (L-686,292) STRUCTURE IV

Microorganism and Culture Conditions

Frozen vegetative mycelium (MA 6559) ATCC No. 53771 was thawed and used to inoculate a 250 ml baffled shake flask containing 50 ml of autoclaved (sterilized) seed medium A consisting of (in units of grams/liter) dextrin 10.0, dextrose 1.0, beef extract 3.0, ardamine PH (Yeast Products, Inc.) 5.0, N-Z Amine type E 5.0, $MgSO_4.7H_20$ 0.05, $KH_2PO_4$ 0.37, and $CaCO_3$ 0.5. The pH of the seed medium was adjusted to 7.1 before autoclaving. The seed was incubated in the seed medium at 27° C for 24 hours on a rotary shaker operating at 220 rpm. A 2.5 ml aliquot of the resulting seed medium was used to inoculate a 250 ml non-baffled shake flask containing 50 ml of the following previously autoclaved (sterilized) transformation medium B. L-683,590 was added as a solution in dimethylsulfoxide to achieve a final concentration of 0.1 mg/ml concentration. The shake flask contents were subsequently incubated for 24 hours at 27° C on a rotary shaker operating at 220 rpm.

1. Transformation medium B consisted of (in grams/liter) glucose 10.0; Hycase SF 2.0; beef extract 1.0; corn steep liquor 3.0; where the pH was adjusted to 7.0 before autoclaving.

Isolation and Purification Procedure for the Broth

The whole broth (500 ml) of transformation media B was extracted three times with methylene chloride (3 x 500 ml). Methylene chloride extracts were combined, dried over sodium sulfate, and concentrated under vacuum to an oily residue. The residue was dissolved in acetonitrile and subjected to high performance liquid chromatography (HPLC) purification.

HPLC was carried out on Whatman Partisil 10 ODS-3, 9.4 mm x 25 cm column and monitored at 205 nm and 225 nm at 60° C. The column was developed at 3 ml./min with linear gradient from 0.1% aqueous $H_3PO_4$-$CH_3CN$, 55:45 to 0.1% aqueous $H_3PO_4$-$CH_3CN$, 20:80 in 60 minutes. The compound was collected during repeated injections of the above described extract. The fractions at retention time 25 minutes were pooled, adjusted to pH 6.5 and evaporated to remove acetonitrile. The compound was further purified using a $C_{18}$ Sep-Pak (Waters Associates) and acetonitrile-water elution solvent to yield 1.2 mg. of product, designated as L-686,292.

Characterization

L-686,292 was characterized via NMR spectrometry which confirms the assigned molecular structure of Structure IV.

EXAMPLE 4

SYNTHESIS OF 13,15,31-TRISDESMETHYL RING REARRANGED FK-900520 (L-687,795) STRUCTURE V

Microorganism and Culture Conditions

Frozen vegetative mycelium (1.0 m.) (MA 6559) ATCC No. 53771 was thawed and used to inoculate a 250 ml baffled shake flask containing 50 ml of autoclaved (sterilized) seed medium A consisting of (in units of grams/liter) dextrin 10.0, dextrose 1.0, beef extract 3.0, ardamine PH (Yeast Products, Inc.) 5.0, N-Z Amine type E 5.0, $MgSO_4.7H_20$ 0.05, $KH_2PO_4$ 0.37, and $CaCO_3$ 0.5. The pH of the seed medium was adjusted to 7.1 before autoclaving. The seed was incubated in the seed medium at 27° C for 24 hours on a rotary shaker operating at 220 rpm. A 2.5 ml aliquot of the resulting seed medium was used to inoculate a 250 ml non-baffled shake flask containing 50 ml of the following previously autoclaved (sterilized) transformation medium B. L-683,590 was added as a solution in dimethylsulfoxide to achieve a final concentration of 0.1 mg/ml concentration. The shake flask contents were subsequently incubated for 24 hours at 27° C on a rotary shaker operating at 220 rpm.

1. Transformation medium B consisted of (in grams/liter) glucose 10.0; Hycase SF 2.0; beef extract 1.0; corn steep liquor 3.0; where the pH was adjusted to 7.0 before autoclaving.

Isolation and Purification Procedure for the Broth

The whole broth (500 ml) of transformation media B was extracted three times with methylene chloride (3 x 500 ml). Methylene chloride extracts were combined, dried over sodium sulfate, and concentrated under vacuum to an oily residue. The residue was dissolved in acetonitrile and subjected to high performance liquid chromatography (HPLC) purification.

HPLC was carried out on Whatman Partisil 10 ODS-3, 9.4 mm x 25 cm column and monitored at 205 nm and 225 nm at 60° C. The column was developed at 3 ml./min with linear gradient from 0.1% aqueous $H_3PO_4$-$CH_3CN$, 55:45 to 0.1% aqueous $H_3PO_4$-$CH_3CN$, 20:80 in 60 minutes. The compound was collected during repeated injections of the above described extract. The fractions at retention time 12.5 minutes were pooled, adjusted to pH 6.5 and evaporated to remove acetonitrile. The compound was further purified using a $C_{18}$ Sep-Pak (Waters Associates) and acetonitrile-water elution solvent to yield 2.5 mg. of product, designated as L-687,795.

Characterization

L-687,795 was characterized via NMR spectrometry which confirms the assigned molecular structure as Structure V.

EXAMPLE 5

Substrate specificity and biosynthetic potential of 31-O-desmethylimmunomycin O-methyltransferase (DIMT) in production of novel immunomycin (FK520) related compounds.

Substrate specificity of a highly purified preparation of 31-O-desmethylimmunomycin O-methyltransferase which had been isolated from S. hygroscopicus (MA 6678-162xp16) was examined by using 13-O-mono-; 13, 31-bis-; 15, 31-bis-; and 13, 15, 31-trisdesmethylimmunomycin as substrates under standard assay condition in which 14-C SAM(S-adenosyl-L-[methyl-14-C]methionine was utilized as methyl donor. By following the radioactivity, products of the enzymic reactions were analyzed by both TLC and HPLC systems. In each case, except 13-desmethylimmunomycin, a single fraction was located which, on the basis of its Rf value on the TLC plate and elution time from HPLC column, was suggestive of the specific methylation at 31-O-position of the respective substrate. There was no reaction when 13-O-desmethylimmunomycin had been used as substrate, however. In order to generate more of the enzymic reaction products toward the goal of the chemical characterization and determination of their biological activities, a large-scale enzyme assay was developed and optimized for each substrate and, in each case, the product was isolated and characterized as follows.

**Biosynthesis of 15-O-desmethylimmunomycin-Structure VII**

15,31-bis-desmethylimmunomycin (Structure IV) was used as substrate after HPLC purification.

**Large-scale enzyme assay.** The reaction mixture contained 35 mg of protein of a partially purified fraction; 2 mM of magnesium sulfate; 6.25 uM substrate; 60 uM SAM in a total volume of 10 ml which was adjusted with 50 mM of phosphate buffer, pH = 7.30. The reaction was initiated by the addition of SAM under incubation conditions as previously reported. The reaction mixture was extracted with ethylacetate and, after work-up, was subjected to a TLC system in which chloroform:methanol (9:1) was used as solvent. The silica gel from the area of the TLC plate, having Rf value that was established from the isolation of radioactivity in a corresponding experiment in which 14-C SAM had been used, was scraped off and eluted extensively with ethylacetate. Ethylacetate extract was then prepared for purification by reverse-phase HPLC. Reverse-phase HPLC system was carried out in a solvent system consisting of water:acetonitrile: phosphoric acid (45: 55: 0.1) at 60 ° C. Fractions eluting with an elution time corresponding to what had already been established for the product of the enzymic assay in which 14-C SAM had been used were collected and pooled. Pooled fractions was then further purified for biological testing and chemical characterization.

Fast Atom Bombardment (FAB) Mass and NMR Spectrometric Analysis. The product of the enzymic reaction as was isolated above was examined by FAB and NMR, confirming the assigned Structure VII, 15-desmethylimmunomycin, with a molecular weight of 777.

**Biosynthesis of 13,15-bis-desmethylimmunomycin (Structure VIII)**

In a series of experiments, 13,15 31-trisdesmethylimmunomycin (Structure V) was used as substrate in an enzymic reaction similar to what had been carried out for the biosynthesis of 15-O-desmethylimmunomycin. The product of this enzymic reaction was similarly isolated as Structure VIII based on TLC and HPLC elution profiles.

**Biosynthesis of 13 (14)-OH-immunomycin (Structure VI)**

In similar experiments as above, 13(14), 31-bisdesmethylimmunomycin (Structure III) was utilized as substrate in an enzymic reaction and the product was isolated. The isolated product has identical Rf value and elution times on TLC and reverse-phase HPLC, respectively, to that of standard 13-(14)-OH-immunomycin Structure VI.

**Biological activities of 15-O-(Structure VII); and 13,15-bis-desmethylimmunomycin (Structure VIII)**

HPLC purified samples of enzymatically made 15-O-desmethylimmunomycin and 13, 15-bisdesmethylimmunomycin were examined for biological activities. The results are shown in Table 1, below.

EXAMPLE 6

T-Cell Proliferation Assay

1. Sample Preparation

Purified samples of L-687,795 (Structure V), Structure VIII, L-616,292 (Structure IV) and Structure VII, as prepared by HPLC above, were independently dissolved in absolute ethanol at 10 mg/ml.

2. Assay

Spleens from C57B1/6 mice were taken under sterile conditions and gently dissociated in ice-cold RPMI 1640 culture medium (GIBCO, Grand Island, N.Y.) supplemented with 10% heat-inactivated fetal calf serum (GIBCO). Cells were pelleted by centrifugation at 1500 rpm for 8 minutes. Contaminating red cells were removed by treating the pellet with ammonium chloride lysing buffer (GIBCO) for 2 minutes at 4°C. Cold medium was added and cells were again centrifuged at 1500 rpm for 8 minutes. T lymphocytes were then isolated by separation of the cell suspension on nylon wool columns as follows: Nylon wool columns were prepared by packing approximately 4 grams of washed and dried nylon wool into 20 ml plastic syringes. The columns were sterilized by autoclaving at 250°F for 30 minutes. Nylon wool columns were wetted with warm (37°C) culture medium and rinsed with the same medium. Washed spleen cells resuspended in warm medium were slowly applied to the nylon wool. The columns were then incubated in an upright position at 37°C for 1 hour. Non-adherent T lymphocytes were eluted from the columns with warm culture medium and the cell suspensions were spun as above.

Purified T lymphocytes were resuspended at 2.5 x $10^5$ cells/ml in complete culture medium composed of RPMI 1640 medium with 10% heat-inactivated fetal calf serum, 100 mM glutamine, 1 mM sodium pyruvate, 2 x $10^{-5}$ M 2-mercaptoethanol and 50 mg/ml gentamycin. Ionomycin was added at 250 ng/ml and PMA at 10 ng/ml. The cell suspension was immediately distributed into 96 well flat-bottom microculture plates (Costar) at 200 µl/well. The control, being the medium without test drug, and various below-indicated dilutions of the sample (above-described purified samples of IV, V, VII, VIII) to be tested were then added in triplicate wells at 20 µl/well. L-683,590 was used as a standard. The culture plates were then incubated at 37°C in a humidified atmosphere of 5% $CO_2$-95% air for 44 hours. The proliferation of T lymphocytes was assessed by measurement of tritiated thymidine incorporation. After 44 hours of culturing, the cells were pulse-labelled with 2 µCi/well of tritiated thymidine (NEN, Cambridge, MA). After another 4 hours of incubation, cultures were harvested on glass fiber filters using a multiple sample harvester. Radioactivity of filter discs corresponding to individual wells was measured by standard liquid scintillation counting methods (Betacounter). Mean counts per minute of replicate wells were calculated and the results expressed as percent inhibition of tritiated thymidine uptake (proliferation) as follows:

$$\% \text{ Inhibition} = 100 - \left[ \frac{\text{Mean cpm sample tested}}{\text{Mean cpm control medium}} \text{ X } 100 \right]$$

The results of % inhibition at various concentrations of the test samples are presented in the following Table 1 showing that Structure IV is a pure antagonist and Structure VII is an antagonist at moderately low dosages but a mixed agonist/antagonist at higher dosages and much lower dosages.

TABLE 1

Effects of 15-desMet-590 and 15,31-desMet-590 on the proliferative response of iono+PMA stimulated T cells

| Sample | Concentration (ng/ml) | % Inhibition No FK-506 | + FK-506 (0.6 nM) |
|---|---|---|---|
| Medium (control) | | 0 | 98 |
| 15-desMet (VII) | 1000 | 100 | 99 |
| | 500 | 74 | 70 |
| | 250 | 56 | 60 |
| | 125 | 42 | 47 |
| | 62 | 41 | 49 |
| | 31 | 43 | 81 |
| | 16 | 9 | 88 |
| | 8 | 5 | 96 |
| 15,31-desMet (IV) | 1000 | 6 | 40 |
| | 500 | 0 | 36 |
| | 250 | 0 | 45 |
| | 125 | 0 | 68 |
| | 62 | 0 | 77 |
| | 31 | 0 | 98 |

+ The proliferative response of ionomycin + PMA activated T cells was measured by the MTT colorimetric assay

According to these data:
-15-desMet-590 (VII) is a mixed agonist/antagonist
and -15,31-desMet-590 (IV) is a pure antagonist

## Claims

1. A composition comprising a cell-free extract of purified enzyme, DIMT, having a molecular weight of about 32 K daltons, as measured by SDS-PAGE, an isoelectric point (PI) of 4.4., and capable of catalyzing the C-31 O-methylation of a C-31 hydroxy containing FK-506 type molecule, in the presence of a methyl transfer agent, when supplemented with Mg $^{+}2$ ion.

2. A process for methylating the C-31 hydroxy group in a C-31 hydroxy-containing FK-506 type molecule comprising the step of contacting the FK-506 type molecule with the methyl transfer agent S-adenosyl methionine in the presence of the DIMT enzyme described in Claim 1, supplemented with the Mg $^{+}2$ ion, in an aqueous solvent therefor.

3. The process of Claim 2 conducted at a pH from 7-9.

4. The process of Claim 2 conducted in the temperature range of 25-40 °C.

**5.** The process of Claim 1 wherein said FK-506 type molecule is of the formula:

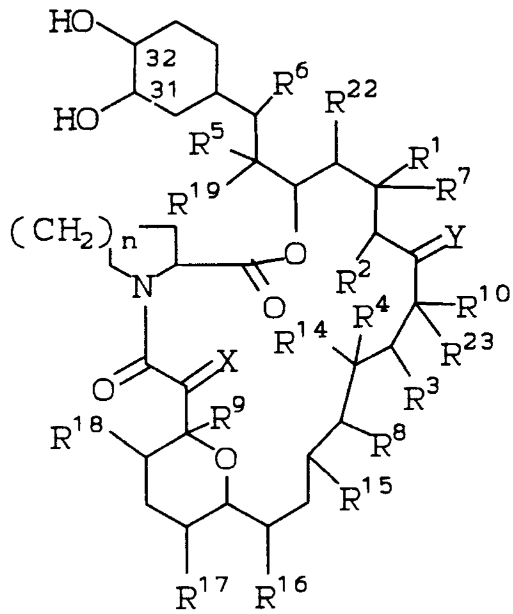

I

wherein each vicinal pair of substituents:
$R^1$ and $R^2$; $R^3$ and $R^4$; $R^5$ and $R^6$; independently:
a) represent two vicinal hydrogen atoms, or
b) form a second bond between the vicinal carbon atoms to which they are attached;
in addition to it significance above,

| | |
|---|---|
| $R^2$ | can represent an $C_1$-$C_{10}$ alkyl group; |
| $R^7$ | represents H, OH or O-$C_1$-$C_{10}$ alkyl, or in conjunction with $R^1$ it may represent =O; |
| $R^8$ and $R^9$ | independently represent H or OH; |
| $R^{10}$ | represents H; $C_1$-$C_{10}$ alkyl, wherein said alkyl can be substituted by one or more hydroxyl groups; $C_1$-$C_{10}$ alkenyl, which can be substituted by one or more hydroxyl groups, or $C_1$-$C_{10}$ alkyl substituted by =O; |
| X | represents O, (H, OH), (H,H) or $-CH_2O-$; |
| Y | represents O, (H, OH), (H,H), N-$NR^{11}R^{12}$ or N-$OR^{13}$ |

wherein,

| | |
|---|---|
| $R^{11}$ and $R^{12}$ | independently represent<br>H,<br>$C_1$-$C_{10}$alkyl,<br>$C_1$-$C_{10}$aryl or tosyl, and |
| $R^{13}$, $R^{14}$, $R^{15}$, $R^{16}$, $R^{17}$, $R^{18}$, $R^{19}$, $R^{22}$ and $R^{23}$ | independently represent<br>H or $C_1$-$C_{10}$ alkyl;<br>n is 1,2, or 3; |

in addition to their significances above, Y, $R^{10}$ and $R^{23}$, together with the carbon atoms to which they are attached, can represent a 5- or 6- membered N-, S- O-containing heterocyclic ring, which is saturated or unsaturated, and which can be substituted by one or more groups selected from $C_1$-$C_{10}$ alkyl, hydroxyl, $C_1$-$C_{10}$ alkyl substituted by one or more hydroxyl groups, O-$C_1$-$C_{10}$ alkyl, benzyl and $-CH_2Se(C_6H_5)$; provided that when X and Y both represent O; $R^9$ represents OH; $R^{14}$, $R^{15}$, $R^{16}$, $R^{17}$, $R^{18}$, $R^{19}$ and $R^{22}$ each represent methyl; $R^8$ and $R^{23}$ each represent H; [$R^3$ and $R^4$] and [$R^5$ and $R^6$] each represent a carbon-carbon bond; and pharmaceutically acceptable salts thereof, which includes acid addition salts of any amine groups present.

6. The process of Claim 5 wherein said FK-506 type compound is of the formula:

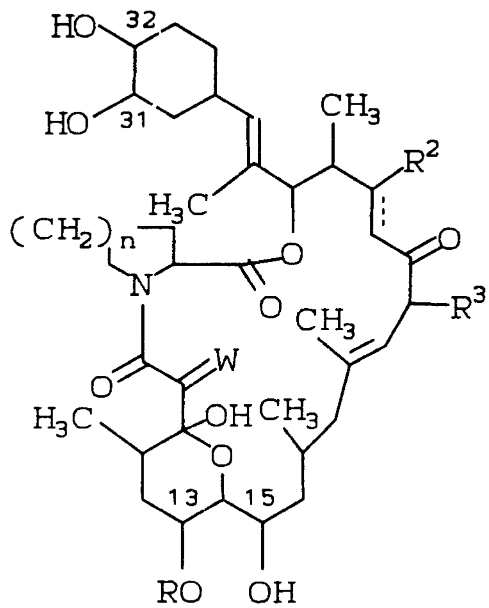

A

wherein:

- W is O, (H, OH), (H, H);
- R is H, $CH_3$;
- $R^2$ is hydrogen, hydroxy or lower alkanoyloxy,
- $R^3$ is methyl, ethyl, propyl or allyl,
- n is an integer of 1 or 2, and the symbol of a line and dotted line is a single bond or a double bond,

and a pharmaceutically acceptable basic salt thereof.

7. The process of Claim 6 wherein said FK-506 type compound is of the formula wherein:
   - R is $CH_3$
   - $R^2$ is hydroxy;
   - $R^3$ is ethyl;
   - n is 2.

8. The process of Claim 6 wherein said FK-506 type compound is of the formula wherein:
   - R is H;
   - $R_2$ is hydroxy;
   - $R_3$ is ethyl;
   - n is 2.

9. The process of Claim 7 wherein said FK-506 type compound is of the formula:

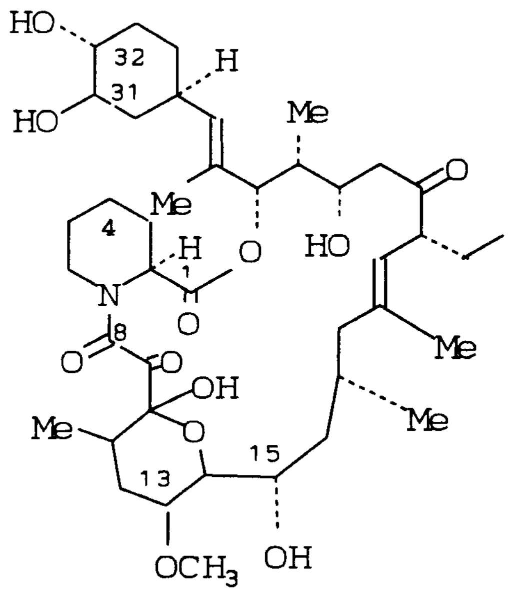

L-686, 292

IV

**10.** The process of Claim 5 wherein said FK-506 type compound is of the formula:

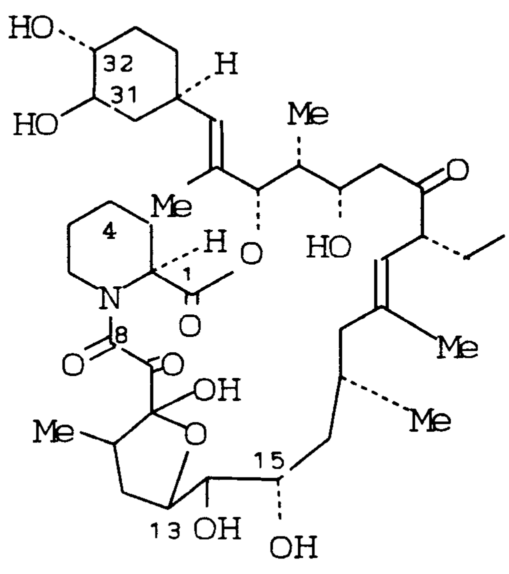

L-687-795

V

**11.** A compound of the formula:

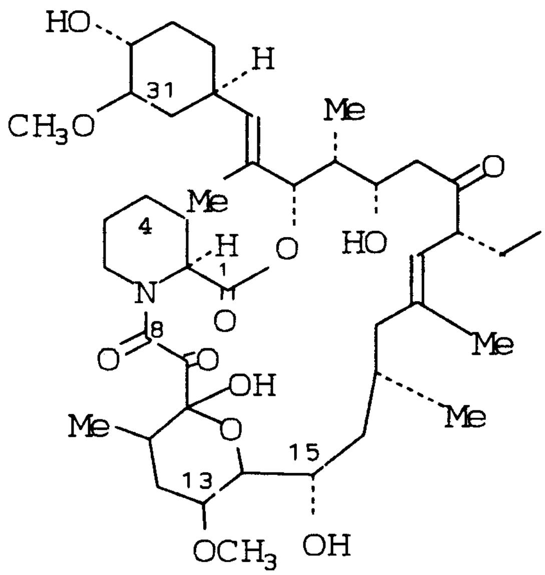

VII

**12.** A Compound of the formula:

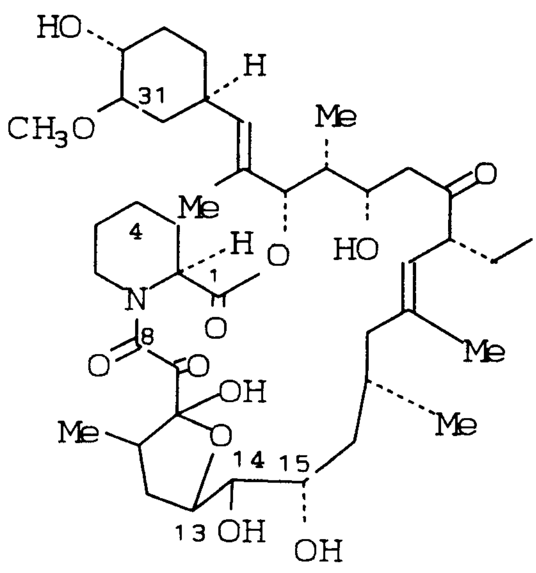

VIII

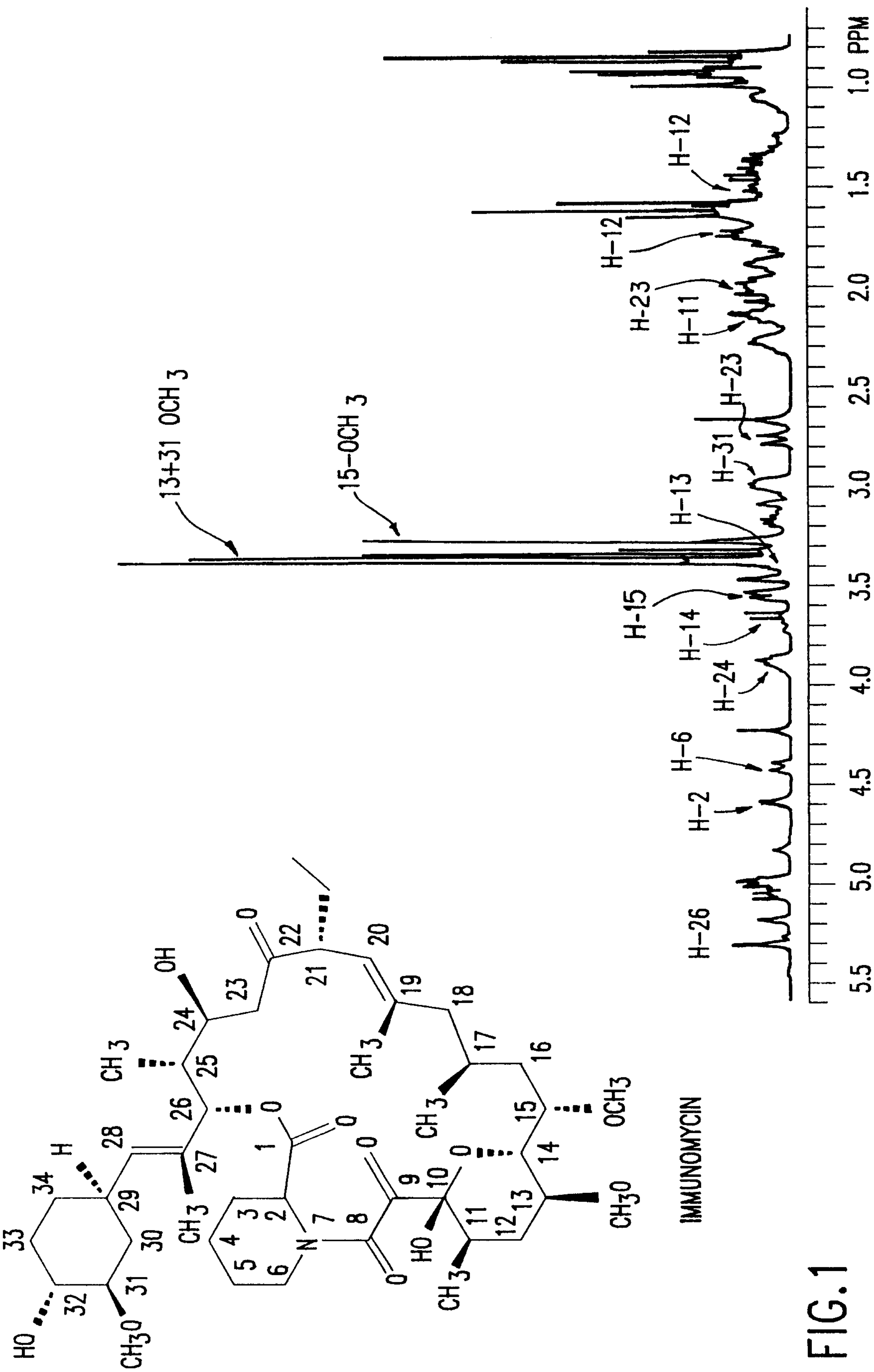


IMMUNOMYCIN

FIG. 1

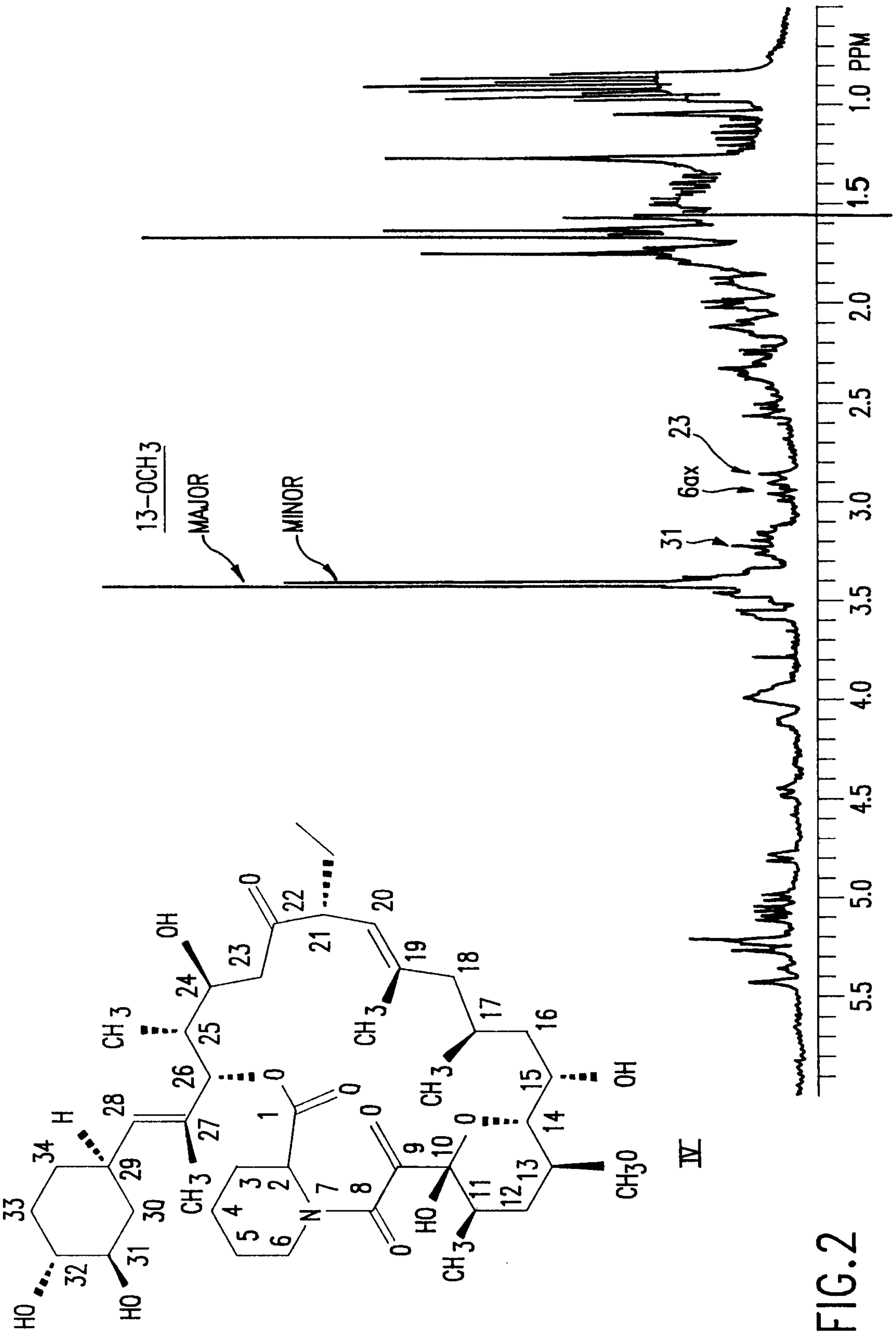


IV

FIG.2

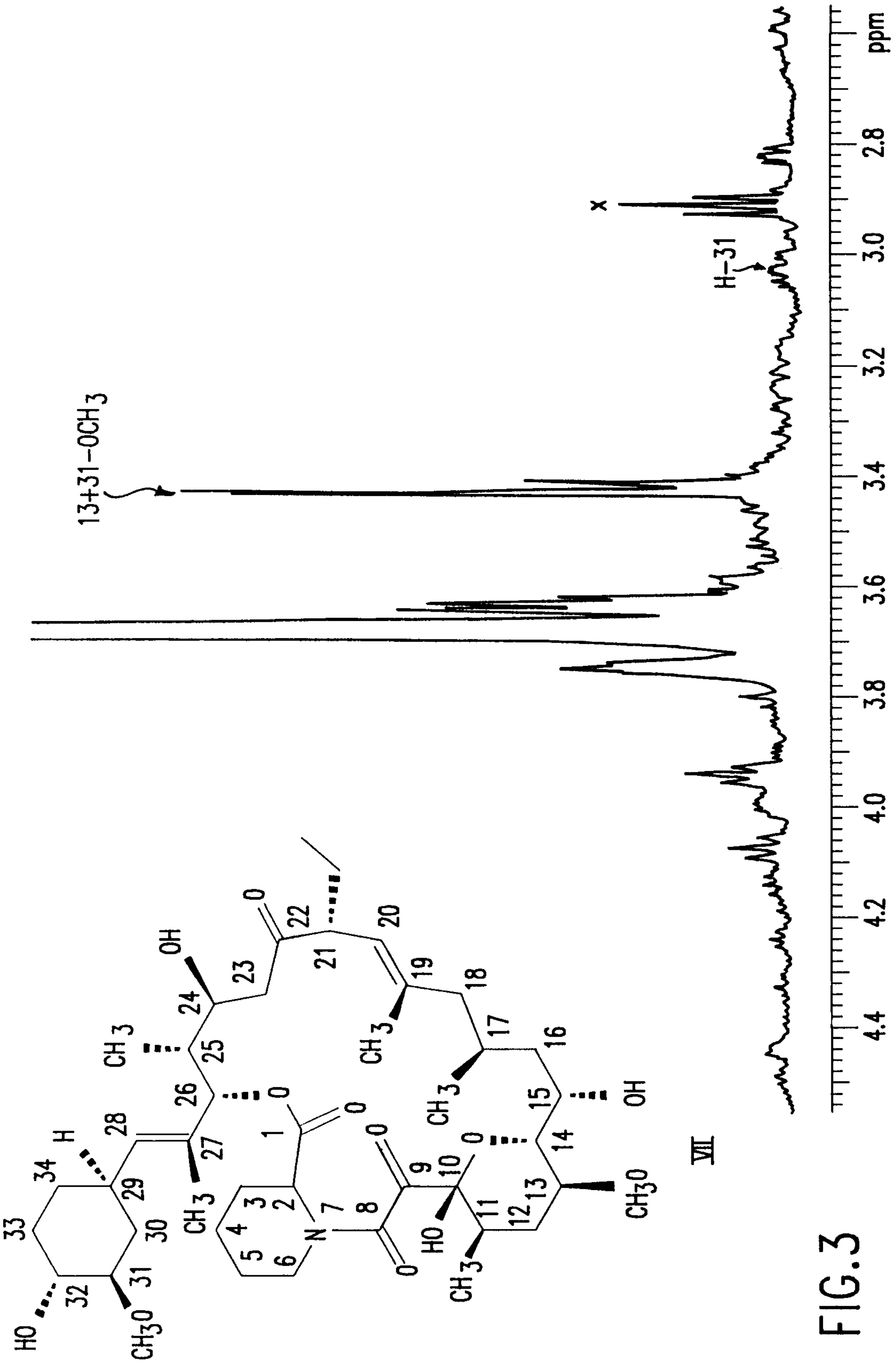


VII

FIG.3

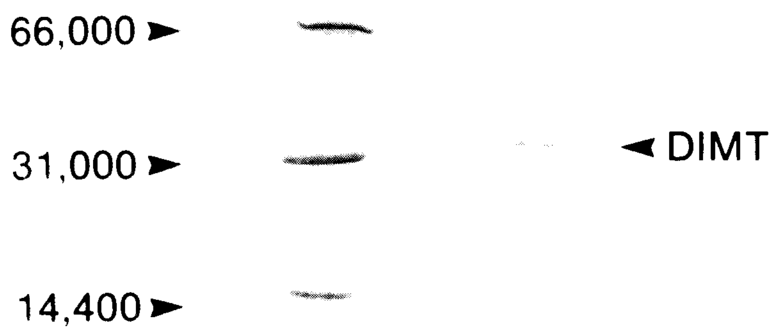


FIG. 4

European Patent Office

# EUROPEAN SEARCH REPORT

Application Number

EP 92 20 1694

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y<br>A | EP-A-0 403 242 (MERCK & CO.INC.)<br>* the whole document *<br>--- | 11,12<br>2,5-10 | C12N9/10<br>C12P17/18<br>C07D498/02 |
| Y<br>A | EP-A-0 378 321 (MERCK & CO.INC.)<br>* the whole document *<br>--- | 11,12<br>2,5-10 | |
| A | EP-A-0 349 061 (MERCK & CO. INC.)<br>* the whole document *<br>--- | 2,5-12 | |
| D,A | EP-A-0 396 399 (MERCK & CO. INC.)<br>* the whole document *<br>--- | 2,5-12 | |
| D,A | US-A-4 894 366 (MASAKUNI OKUHARA ET AL.)<br>* the whole document *<br>--- | 2,5-12 | |
| A | EP-A-0 391 594 (MERCK & CO. LTD.)<br>* page 3, line 1 - page 6, line 42 *<br>--- | 1,2 | |
| D,A | EP-A-0 323 865 (MERCK & CO.INC.)<br>* the whole document *<br>----- | 1,2 | |
| | | | **TECHNICAL FIELDS SEARCHED (Int. Cl.5)** |
| | | | C12N<br>C12P<br>C07H |

The present search report has been drawn up for all claims

| Place of search | Date of completion of the search | Examiner |
|---|---|---|
| BERLIN | 20 OCTOBER 1992 | GURDJIAN D. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document